# EUROPEAN PATENT APPLICATION

(11) **EP 2 201 946 A1**
(43) Date of publication of application: **30.06.2010**
(21) Application number: 08842108.6
(22) Date of filing: 23.10.2008
(51) Int. Cl.: A61K 31/167, A61K 31/5375, A61P 7/02, A61P 43/00, C07D 295/20

(54) **PAI-1 PRODUCTION INHIBITOR**

(30) Priority: 23.10.2007 JP 2007275557
(71) Applicant: Institute of Medicinal Molecular Design, Inc., Tokyo 113-0033 (JP)
(72) Inventor: MUTO, Susumu, Tokyo 113-0033 (JP); ITAI, Akiko, Tokyo 113-0033 (JP)
(74) Representative: Polypatent
(86) International application number: PCT/JP2008/069207
(87) International publication number: WO 2009/054439

(57) **Abstract**

The compound represented by the following general formula (I) has an inhibitory activity on PAI-1 production; 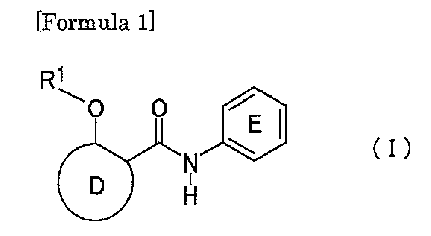 wherein R¹ represents, for example, a hydrogen atom, or a 4-(morpholinyl)carlaonyl group, ring D represents, for example, a benzene ring or a benzene ring having substituent(s), and phenyl group E has substituent(s) such as a halogen atom, a halogenated alkyl group, an alkyl group, a halogenated alkoxy group, or an alkoxy group.

## Description

The present invention relates to uses of substances that inhibit PAI-1 production.

### [Background Art]

It is known that an overexpression of plasminogen activator inhibitor-1 (PAI-1) inhibits the production of plasmin that decomposes fibrin thrombi and tissue proteins and brings a formation of thrombi (Nature, Vol. 346, No.6279, pp.74-76, 1990) by inhibiting the activation of plasminogen activator (for example, tissue-type plasminogen activator and urokinase-type plasminogen activator).
Furthermore, the overexpression of PAI-1 is observed in arteriosclerotic lesions, and is known to increase a risk of thrombotic diseases such as cardiac infarction, deep-vein thrombosis (DVT), disseminated intravascular coagulation (DIC) following sepsis and the like (Proc. Natl. Acad. Sci. U. S. A., Vol.89, No.15, pp.6998-7002, 1992).
From these facts, compounds that inhibit PAI-1 activity or production are useful for the suppression of forming thrombus, and are expected to be useful drugs to diseases caused by the formation of thrombi such as thrombotic diseases, diseases involving thrombus formation and the like.

Furthermore, it is known that PAI-1 promotes precipitation and accumulation of extracellular matrix and is deeply involved in the development of tissue lesion featuring fibrosis and vascular wall sclerotic lesion. As an extracellular matrix accumulation in the airway of an asthma model mouse is reduced by a PAI-1 knockout (Biochem. Biophys. Res. Common., Vol.294, No.5, pp.1155-1160, 2002), it is suggested that PAI-1 is directly involved in the fibrosis. Therefore, compounds that inhibit the activity or production of PAI-1 are useful for the inhibition of fibrosis of tissue, and are expected to be useful drugs to diseases caused by the fibrosis of tissue.

Besides, PAI-1 is also secreted from mast cells (J. Immunol., Vol.165, No.6, pp.3154-3161, 2000), and it is reported that the blood level is high in an obesity model mouse, and is synthesized not only in the endothelial tissue and in the hepatic tissue, but also synthesized in the fatty tissue. Particularly in the visceral fat, PAI-1 synthesis amount is exponentially enhanced together with its deposition (Mol. Med., Vol.2, No. 5, pp.568-582, 1996). Moreover, in the obesity model mouse where PAI-1 gene is knocked out, a decrease of weight and a lowering of blood glucose level and blood insulin level are reported (FASEB J., Vol. 15, No. 10, pp.1840-1842, 2001), and it shows that PAI-1 has a possibility to aggravate various pathological conditions caused by the fat deposition. Therefore, compounds that inhibit the activity or the production of PAI-1 are useful for the inhibition of visceral fat deposition, and are expected to be useful drugs to diseases caused by the visceral fat deposition.

Furthermore, from the facts that PAI-1 inhibits an adhesion of cell and extracellular matrix by binding to a vitronection which is a cell adhesion factor, a PAI-1 antibody inhibits cancer metastasis in a cancer model (Gen. Diagn. Pathol., Vol.141, No.1, pp.41-48, 1995), and the infiltration of cancer and angiogenesis are inhibited when malignant keratinocytes are transplanted to the PAI-1 knockout mouse (Nat. Med., Vol.4, No.8, pp.923-928, 1998), compounds that inhibit the activity or the production of PAI-1 are useful for the inhibition of cell migration, cell metastasis, angiogenesis and the like, and are expected to have therapeutic effects to diseases caused by cell migration, cell metastasis, angiogenesis and the like.

Moreover, arterial lesions as an acute rejection and a chronic rejection after heart or kidney transplantation are considered to be caused by the development of tissue fibrosis, the formation of thrombi, and the proliferation and the reconstruction of arterial endothelial cell. Since, in the heart transplantation experiment using mice (murine), when a compound having an inhibitory activity to PAI-1 is administered, a graft survival is significantly prolonged compared with the control group and the rate of serious intimal hypertrophy is reduced to one third (European Patent Application Publication No.1666469 Specification), compounds that inhibit the activity or the production of PAI-1 are expected as a medicament to inhibit an acute rejection and an arterial lesion after transplantation, after heart or kidney transplantation or transplantation of other organs.

On the other hand, as plasmin which is activated by inhibiting PAI-1 is involved not only in the decomposition of thrombi, but also in the reconstruction of tissues, the migration, metastasis and infiltration of cells, the ovulation and implantation, the activation of transforming growth factors which are cytostatic cytokines, and the activation of collagenase, compounds that inhibit the activity or the production of PAI-1 are useful for the inhibition of tissue reconstruction, the proliferation, migration, infiltration and metastasis of cells, angiogenesis and the like, and treatment effects are expected to diseases caused by cell proliferation, angiogenesis, reconstruction of tissues and the like.

In the past, various compounds have been reported as the substances having PAI-1 inhibition activity (European Patent Application Publication No. 1666469 Specification; International Publication No. 95/32190 Pamphlet; International Publication No. 95/21832 Pamphlet; U. K. Patent Application Publication No.2372740 Specification; International Publication No 03/000253 Pamphlet; International Publication No 03/000258 Pamphlet; International Publication No 03/000649Pamphlet International Publication No 03/000671 Pamphlet; International Publication No 03/000684 Pamphlet; International Publication No 2004/052856 Pamphlet; International Publication No 2004/052893 Pamphlet; International Publication No 2005/030192 Pamphlet; International Publication No 2005/030204 Pamphlet; International Publication No 2005/030715 Pamphlet; International Publication No 2005/030716 Pamphlet; International Publication No 2005/030756 Pamphlet; U.S. Patent Application Publication No. 2005/0124664 Specification; U.S. Patent Application Publication No 2005/0124667 Specification; U.S. Patent Application Publication No 2005/0143384 Specification; Biochemistry, Vol.37, No.5, pp.1227-1234, 1998; J. Med Chem., Vol.47, No.14, pp.3491-3494, 2004). Moreover, examples of inhibiting substance of gene expression of PAI-1 induced by PMA (phorbol 12-myristate 13-acetate) include cyclosporine and cycloheximide (J. Immunol., Vol.165, pp.3154-3161, 2000).

However, it has been reported that PAI-1 (Plasminogen activator inhibitor-1) gene expression induced by PMA (phorbol 12-myristate 13-acetate) was not inhibited by dexamethasone that is a steroid drug (J. Immunol., Vol.165, No.6, pp.3154-3161, 2000). Moreover, it has been reported that PAI-1 production induced by TGF-β and TNF-α increased by dexamethasone (Comp. Hepatol., 2007 May 2;6:5 and Pathobiology, vol.71, pp.295-301, 2004).

### [Disclosure of the Invention]

### [Problems to be solved by the Invention]

An object of the present invention is to provide novel substances that inhibit PAI-1 production and are useful for inhibiting blood coagulation or thrombus formation, and uses thereof.

### [Means to solve the Problems]

The inventors of the present invention conducted various studies to solve the aforementioned problems and, found as a result, that the compound represented by the following general formula (I) had an inhibitory activity on PAI-1 production, and achieved the present invention. 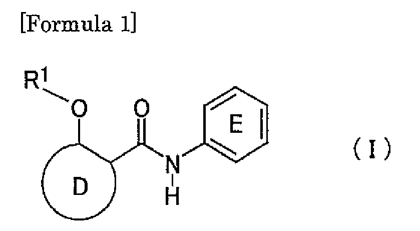 In the formula (I), R¹ represents a hydrogen atom, an alkylcarbonyl group, or a 4-(morpholinyl)carbonyl group;
ring D represents a benzene ring, or a benzene ring having one or more groups selected from the group consisting of a halogen atom, a nitro group, a cyano group, an alkyl group, an alkoxyiminoalkyl group, a benzyloxyiminoalkyl group, a phenylalkenyl group, a phenylalkenyl group, a trimethylsilylalkynyl group, a phenyl group, a phenylalkyl group, a halogenated alkyl group, a pyrrolyl group, an alkylpyrrolyl group, a thienyl group, an alkylthienyl group, an alkylthiazolyl group, a pyridyl group, an alkylpyridyl group, an alkylcarbonyl group, an alkoxycarbonyl group, a pyrrolylsulfonyl group, a (4-nitrophenyl)diazenyl group and a hydroxyl group; and
phenyl group E is a phenyl group substituted by one or more groups selected from the group consisting of a halogen atom, a halogenated alkyl group, a phenyl group, an alkyl group, a halogenated alkoxy group, a phenylalkyl group, an alkoxy group, a cyclohexyl group, a phenylalkoxy group, a cyano group, a halogenated alkylsulfonyl group, a halogenated alkylsulfonyl group, an ethynyl group, a benzoyl group, an alkylsulfanyl group, an alkylsulfonyl group, a morpholino group, an alkylfuryl group, an alkylcarbonyl group, a 1-(5-phenyl)-3-(halogenated alkyl)pyrazolyl group, a 1-(5-alkyl)-3-(halogenated alkyl)pyrazolyl group, and a 1-[3,5-bis(halogenated alkyl)]pyrazolyl group.

The present invention thus includes:
(a) an inhibitor of PAI-1 production which comprises, as an active ingredient, a substance selected from the group consisting of a compound represented by the general formula (I); a salt thereof,; hydrates thereof and solvates thereof;
(b) the inhibitor of PAI-1 production according to (a), wherein R¹ is a hydrogen atom, a C₁₋₆ alkylcarbonyl group, or a 4-(morphonyl)carbonyl group, D is a benzene ring, or a benzene ring having one or more groups selected from the group consisting of a halogen atom, a nitro group, a cyano group, a C₁₋₁₀ alkyl group, a C₁₋₆ alkoxyiminoC₁₋₆ alkyl group, a benzyloxyiminoC₁₋₆ alkyl group, a phenylC₂₋₆ alkenyl group, a phenylC₂₋₆ alkynyl group, a trimethylsilylC₂₋₆ alkynyl group, a phenyl group, a phenyl C₁₋₆ alkyl group, a halogenated C₁₋₆ alkyl group, a pyrrolyl group, a C₁₋₆ alkylpyrrolyl group, a thienyl group, a C₁₋₆ alkylthienyl group, a C₁₋₆ alkylthiazolyl group, a pyridyl group, a C₁₋₆ alkylpyridyl group, a C₁₋₆ alkylcarbonyl group, a C₁₋₆ alkoxycarbonyl group, a pyrrolylsulfonyl group, a (4-nitrophenyDdiazenyl group, and a hydroxyl group, E is a phenyl group substituted by one or more groups selected from the group consisting of a halogen atom, a halogenated C₁₋₆ alkyl group, a phenyl group, a C₁₋₆ alkyl group, a halogenated C₁₋₆ alkoxy group, a phenyl C₁₋₆ alkyl group, a C₁₋₆ alkoxy group, a cyclohexyl group, a phenyl C₁₋₆ alkoxy group, a cyano group, a halogenated C₁₋₆ alkylsulfanyl group, a halogenated C₁₋₆ alkylsulfonyl group, an ethynyl group, a benzoyl group, a C₁₋₆ alkylsulfanyl group, a C₁₋₆ alkylsulfonyl group, a morpholino group, a C₁₋₆ alkylfuryl group, a C₁₋₆ alkylcarbonyl group, a 1-(5-phenyl)-3-(halogenated C₁₋₆ alkyl) pyrazolyl group, a 1-(5- C₁₋₆ alkyl)-3-(halogenated C₁₋₆ alkyl)pyrazolyl group, and a 1-[3,5-bis(halogenated C₁-₆ alkyl)]pyrazolyl group;
(c) the inhibitor of PAI-1 production according to (a), wherein the compound represented by the general formula (I) is a compound represented by any one of the following formulas (1) to (3) (hereinafter, referred to as "compound (1)", "compound (2)", and "compound (3)", respectively); 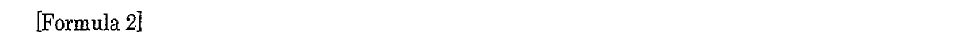 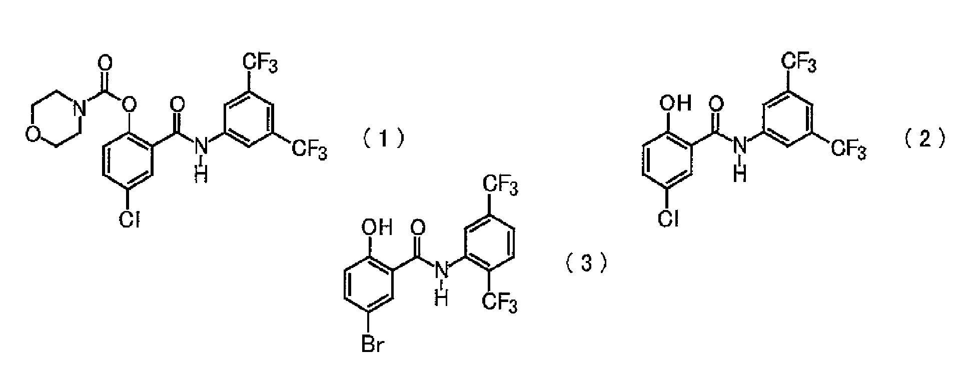
(d) a medicament for prevention or improvement of a disease caused by an overproduction of PAI-1, which comprises, as an active ingredient, the substance according to any one of (a) to (c);
(e) a method of inhibiting PAI-1 production, comprising the step of allowing the substance according to any one of (a) to (c) to act on a PAI-1 producing cell;
(f) a method for prevention or improvement of a disease caused by an overproduction of PAI-1, comprising the step of administering an effective dose of the substance according to any one of (a) to (c) to a mammal including a human;
(g) use of the substance according to any one of (a) to (c) for the manufacture of an inhibitor of PAI-1 production; and
(h) use of the substance according to any one of (a) to (c) for the manufacture of a medicament for prevention or improvement of a disease caused by an overproduction of PAI-1.

### [Brief Explanation of Drawing]

[Figure 1] In one example of the present invention, it is a figure that indicates the inhibitory effect of the compound (1) on PAI-1 production.
[Figure 2] In one example of the present invention, it is a figure that indicates the inhibitory effect of the compound (1) on collagen accumulation.
[Figure3] In one example of the present invention, it is a figure that indicates the results of the changes in the ratio of PAI-1 expression cells to the total number of the infiltration cells when the compound (1) was administered to the guinea pigs exposed to cigarette smoke.

### [Best Embodiment to carry out Invention]

In the following, embodiments of the present invention completed based on the above findings are explained in detail, giving examples. In case no specific explanations are given in the examples, the reagent kits and the measuring devices are used according to the attached protocols.
The purpose, characteristics, advantages, and the idea of the present invention are clear to those skilled in the art by the description of the specification of the present invention, and those skilled in the art can easily reproduce the present invention according to the description of the present specification. The embodiment and the specific examples described below show preferable embodiment of the present invention. They are shown for exemplification or explanation, and the present invention is not limited to them. Within the intention and the scope of the present invention disclosed in the present specification, based on the description of the present specification, it is clear for those skilled in the art to be able to conduct various alterations and modifications.

Examples of substances used in the present invention include a compound represented by the general formula (I) or a salt thereof, a hydrate of the aforementioned compound or the salt thereof, or a solvate of the aforementioned compound or the salt thereof. Moreover, examples of the compound represented by the general formula (I) include the compound represented by the following general formula (II).

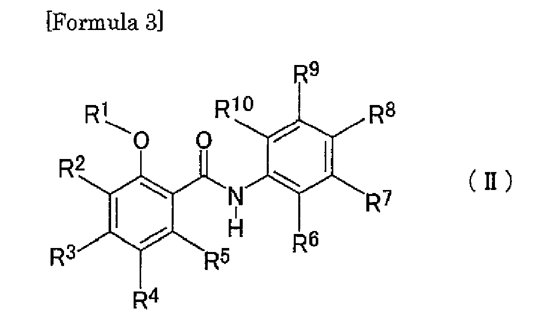

In the present invention, R¹ in the general formula (I) or (II) represents a hydrogen atom, an alkylcarbonyl group, or a 4-(morpholinyl)carbonyl group. It is preferred that R¹ in the general formula (I) or (II) is a hydrogen atom, an acetyl group, or a 4-(morpholinyl)carbonyl group. Moreover, ring D in the general formula (I) represents a benzene ring, or a benzene ring having at least one or more halogen atoms, nitro groups, cyano groups, alkyl groups, alkoxyiminoalkyl groups, benzyloxyiminoalkyl groups, phenylalkenyl groups, phenylalkynyl groups, trimethylsilylalkynyl groups, phenyl groups, phenylalkyl groups, halogenated alkyl groups, pyrrolyl groups, alkylpyrrolyl groups, thienyl groups, alkylthienyl groups, alkylthiazolyl groups, pyridyl groups, alkylpyridyl groups, alkylcarbonyl groups, alkoxycarbonyl groups, pyrrolylsulfonyl groups, (4-nitrophenyl)diazenyl groups or hydroxyl groups. It is preferred that ring D in the general formula(1) is a benzene ring, or a benzene ring having at least one or more halogen atoms, nitro groups, cyano groups, methyl groups, butyl groups (for example tert-butyl groups), octyl groups (for example 1,1,3,3,-tetramethylbutyl groups), propyl groups (for example isopropyl groups), methoxyiminoethyl groups (for example 1-(methoxyimino)ethyl groups), benzyloxyiminoethyl groups (for example 1-[(benzyloxy)imino]ethyl groups), styryl groups, phenylethynyl groups, (trimethylsilyl)ethynyl groups, phenyl groups, phenylethyl groups (for example 2-phenylethyl groups), trifluoromethyl groups, pentafluoroethyl groups, pyrrolyl groups (for example 1-pyrrolyl groups), thienyl groups (for example 2-thienyl groups or 3-thienyl groups), methylthiazolyl groups (for example 2-methylthiazol-4-yl groups), pyridyl groups (for example 2-pyridyl groups), acetyl groups, isobutyryl groups, methoxycarbonyl groups, pyrrolylsulfonyl groups (for example (pyrrol-1-yl)sulfonyl groups), (4-nitrophenyl)diazenyl groups, or hydroxyl groups.

Phenyl group E in the general formula (I) has at least one or more halogen atoms, halogenated alkyl groups, phenyl groups, alkyl groups, halogenated alkoxy groups, phenylalkyl groups, alkoxy groups, cyclohexyl groups, phenylalkoxy groups, cyano groups, halogenated alkylsulfanyl groups, halogenated alkylsulfonyl groups, ethynyl groups, benzoyl groups, alkylsulfanyl groups, alkylsulfonyl groups, morpholino groups, alkylfuryl groups, alkylcarbonyl groups, 1-(5-phenyl)-3-(halogenated alkyl)pyrazolyl groups, 1-(5-alkyl)-3-(halogenated alkyl)pyrazolyl groups, or 1-[3,5-bis(halogenated alkyl)]pyrazolyl group. It is preferred that phenyl group E in the general formula (1) has one or more of halogen atoms, trifluorometyl groups, 2-chloro-1,1,1,3,3,3-hexafluoropropyl groups, phenyl groups, methyl groups, butyl groups (for example n-butyl groups, tert-butyl groups or see-butyl groups), hexyl groups (for example n-hexyl groups), propyl groups (for example isopropyl groups), 2,2,2-tirfluoroethoxy groups, trifluoromethoxy groups, difluoromethoxy groups, benzyl groups, methoxy groups, hexyloxy groups, cyclohexyl groups, benzyloxy groups, cyano groups, trifluoromethylsulfanyl groups, trifluoromethylsulfonyl groups, ethynyl groups, benzoyl groups, methylsulfanyl groups, methylsulfonyl groups, morpholino groups, methylfuryl groups (for example 5-methylfuran-2-yl groups), butylcarbonyl groups (for example tert-butylcarbonyl groups), 1-(5-phenyl)3-(trifluoromethyl)pyrazolyl groups, 1-(5-tert-butyl)-3-(trifluoromethyl)pyrazolyl groups, or 1-[3,5-bis(trifluoromethyl)]pyrazolyl groups.

R², R³, R⁴, and R⁵ in the general formula (II) independently represent hydrogen atom, halogen atom, nitro group, cyano group, alkyl group, alkoxyiminoalkyl group, benzyloxyiminoalkyl group, phenylalkenyl group, phenylalkynyl group, trimethylsilylalkynyl group, phenyl group, phenylalkyl group, halogenated alkyl group, pyrrolyl group, alkylpyrrolyl group, thienyl group, alkylthienyl group, alkylthiazolyl group, pyridyl group, alkylpyridyl group, alkylcarbonyl group, alkoxycarbonyl group, pyrrolylsulfonyl group, (4-nitrophenyl)diazenyl group, or hydroxyl group. It is preferred that R², R³, R⁴, and R⁵ in the general formula (II) is independently hydrogen atom, halogen atom, nitro group, cyano group, methyl group, butyl group (for example tert-butyl group), octyl group (for example 1,1,3,3-tetramethylbutyl group), propyl group (for example isopropyl group), methoxyiminoethyl group (for example 1-(methoxyimino)ethyl group), benzyloxyiminoethyl group (for example 1-[(benzyloxy)imino]etyl group), styryl group, phenylethynyl group, (trimethylsilyl)ethynyl group, phenyl group, phenylethyl group (for example 2-phenylethyl group), trifluoromethyl group, pentafluoroethyl group, pyrrolyl group (for example 1-pyrrolyl group), thienyl group (for example 2-thienyl group or 3-thienyl group), methylthiazolyl group (for example 2-methylthiazol-4-yl group), pyridyl group (for example 2-pyridyl group), acetyl group, isobutyryl group, methoxycarbonyl group, pyrrolylsulfonyl group (for example (pyrrol-1-yl)sulfonyl group), (4-nitrophenyl)diazenyl group, or hydroxyl group.

R⁶, R⁷, R⁸, R⁹, and R¹⁰ in the general formula (II) independently represent hydrogen atom, halogen atom, halogenated alkyl group, phenyl group, alkyl group, halogenated alkoxy group, phenylalkyl group, alkoxy group, cyclohexyl group, phenylalkoxy group, cyano group, halogenated alkylsulfanyl group, halogenated alkylsulfonyl group, ethynyl group, benzoyl group, alkylsulfanyl group, alkylsulfonyl group, morpholino group, alkylfuryl group, alkylcarbonyl group, 1-(5-phenyl)-3-(halogenated alkyl)pyrazolyl group, 1-(5-alkyl)-3-(halogenated alkyl)pyrazolyl group, or 1-[3,5-bis(halogenated alkyl)]pyrazolyl group. It is preferred that R⁶, R⁷, R⁸, R⁹, and R¹⁰ in the general formula (II) is independently hydrogen atom, halogen atom, trifluoromethyl group, 2-chloro-1,1,1,3,3,3-hexafluoropropyl group, phenyl group, methyl group, butyl group (for example n-butyl group, tert-butyl group or sec-butyl group), hexyl group (for example n-hexyl group), propyl group (for example isopropyl group), 2,2,2-trifluoroethoxy group, trifluoromethoxy group, difluoromethoxy group, benzyl group, methoxy group, hexyloxy group, cyclohexyl group, benzyloxy group, cyano group, trifluoromethylsulfanyl group, trifluoromethylsulfonyl group, ethynyl group, benzoyl group, methylsulfanyl group, methylsulfonyl group, morpholino group, methylfuryl group (for example 5-methylfuran-2-yl group), butylcarbonyl (for example tert-butylcarbonyl group), 1-(5-phenyl)3-(trifluoromethyl)pyrazolyl group, 1-(5-tert-butyl)-3-(trifluoromethyl)pyrazolyl groups, or 1-[3,5-bis(trifluoromethyl)]pyrazolyl group. However, it makes it a condition that not all of the R⁶, R⁷, R⁸, R⁹, and R¹⁰ in the general formula (II) are hydrogen atoms.

In the present specification, examples of the halogen atom include, for example, fluorine atom, chlorine atom, bromine atom, and iodine atom.
Examples of the alkyl group include, for example, the following C₁₋₁₅ straight chain or branched chain alkyl groups, a C₁₋₁₀ alkyl groups is preferred, and a C₁₋₆ alkyl group is especially preferred.

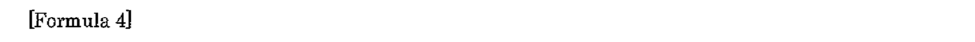

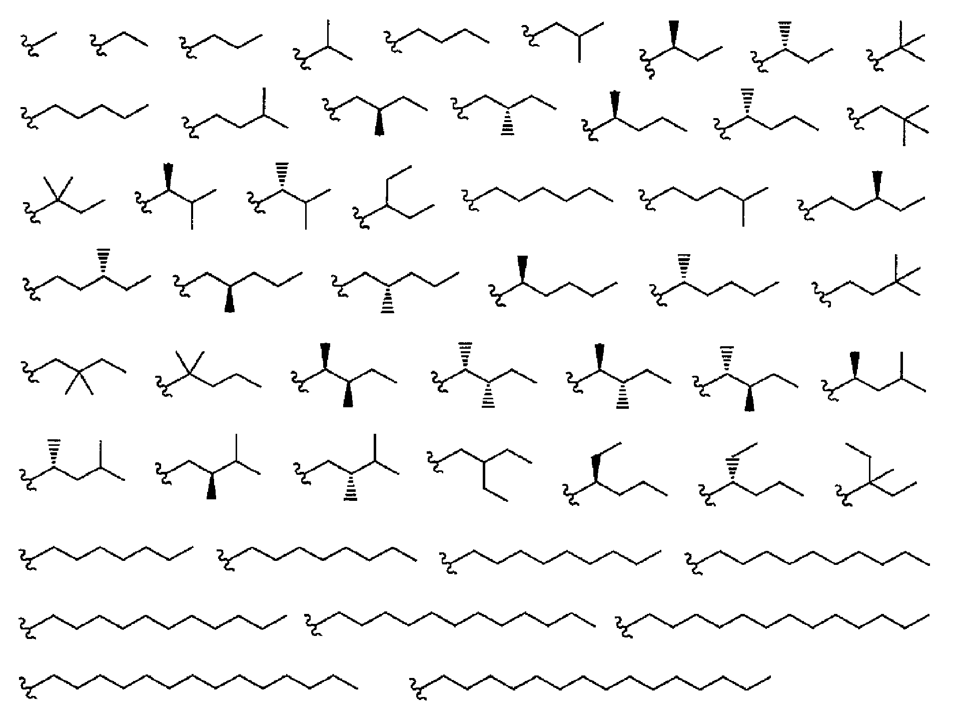

Examples of the alkoxy group (alkyloxy group) include, for example, C₁₋₁₅ straight chain or branched chain alkyloxy groups, a C₁₋₁₀ alkyl groups is preferred, and a C₁₋₆ alkyloxy group is especially preferred.
Examples of the alkenyl group include, for example, the following C₂₋₁₅ straight chain or branched chain alkenyl groups, and a C₂₋₇ alkenyl group is preferred.

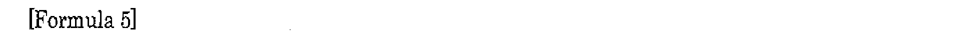

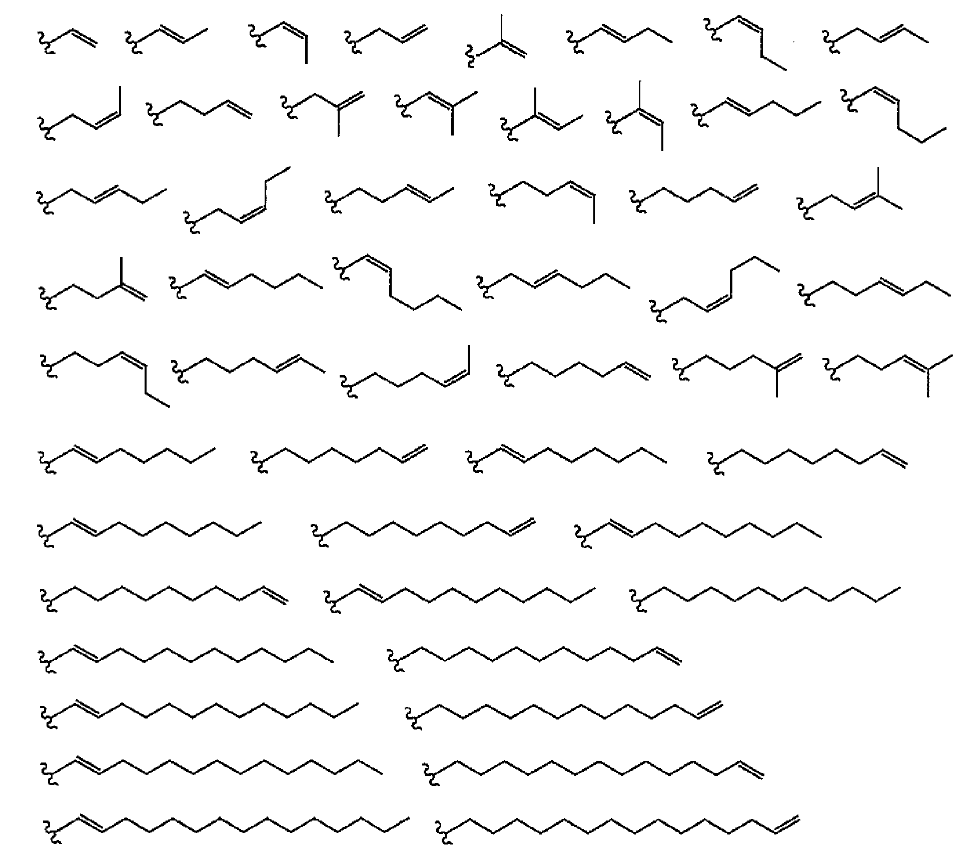

Examples of the alkynyl group include, for example, the following C₂₋₁₅ straight chain or branched chain alkynyl groups, and a C₂₋₇ alkynyl group is preferred.

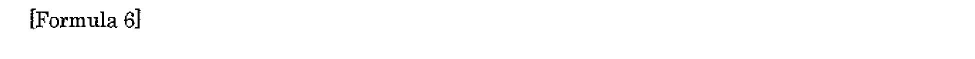

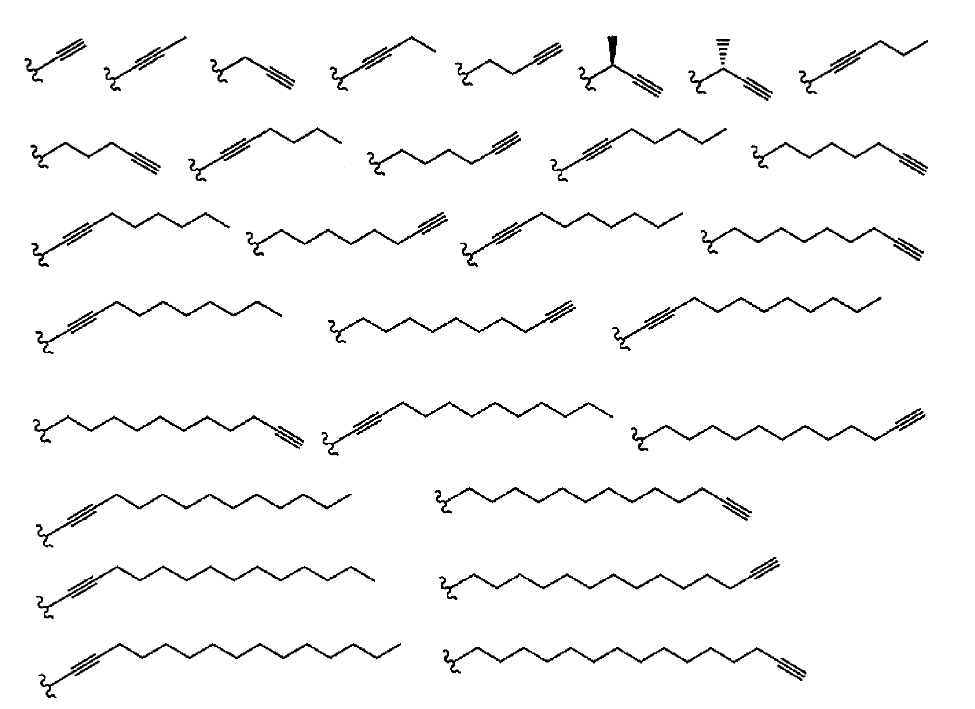

The alkylcarbonyl group includes, for example, a C₁₋₆ alkyl carbonyl group. Moreover, the C₁₋₆ alkylcarbonyl group includes, for example, an acetyl group, an isobutyryl group, or a butylcarbonyl group.
The alkylsulfanyl group includes, for example, a C₁₋₆ alkylsulfanyl group. Moreover, the C₁₋₆ alkylsulfanyl group includes, for example, a methylsulfanyl group.
The alkylsulfonyl group includes, for example, a C₁₋₆ alkylsulfonyl group. Moreover, the C₁₋₆ alkylsulfonyl group includes, for example, a methylsulfonyl group.

The alkylfuryl group includes, for example, a C₁₋₆ alkylfuryl group. Moreover, the C₁₋₆ alkylfuryl group includes, for example, a 2-(5-methyl)furyl group.
The alkylpyrrolyl group includes, for example, a 1-, 2-, or 3-alkylpyrrolyl group. The alkylthienyl group includes, for example, a 2- or 3-alkylthienyl group.
The alkylthiazolyl group includes, for example, a 2-, 4-, or 5-alkylthiazolyl group. The 2-alkylthiazolyl group includes, for example, a 2-C₁₋₆ alkylthiazolyl group. Moreover, the 2-C₁₋₆ alkylthiazolyl group includes, for example, a 2-methylthiazol-4-yl group. The 4-alkylthiazolyl group includes, for example, a 4-C₁₋₆ alkylthiazolyl group. Furthermore, the 4-C₁₋₆ alkylthiazolyl group includes, for example, a 4-methylthiazol-4-yl group. The 5-alkylthiazolyl group includes, for example, a 5-C₁₋₆ alkylthiazolyl group. Furthermore, the 5-C₁₋₆ alkylthiazolyl group includes, for example, a 5-methylthiazol-4-yl group.
The alkylpyridyl group includes, for example, a 2-, 3-, or 4-alkylpyridyl group.

The halogenated alkyl group means an alkyl group in which one or more hydrogen atoms in the alkyl group are substituted by halogen atoms. Examples of the halogenated alkyl group include, for example, a halogenated C₁₋₆ alkyl group. Moreover, the halogenated C₁₋₆ alkyl group includes, for example, a trifluoromethyl group, a 1-chloroethyl group, a pentafluoroethyl group, and a 2-chloro-1,1,1,3,3,3-hexafluoropropyl group.
The halogenated alkoxy group means an alkoxy group in which one or more hydrogen atoms in the alkoxy group are substituted by halogen atoms. Examples of the halogenated alkoxy group include, for example, a halogenated C₁₋₆ alkoxy group. Moreover, the halogenated C₁₋₆ alkoxy group includes, for example, a trifluoromethoxy group, a difluoromethoxy group, and a 2,2,2-trifluoroethoxy group.
The halogenated alkylsulfanyl group means an alkylsulfonyl group in which one or more halogen atoms in the alkylsulfanyl group are substituted by halogen atoms. Examples of the halogenated alkylsulfanyl group include, for example, a halogenated C₁₋₆ alkylsulfanyl group. Moreover, the halogenated C₁₋₆ alkylsulfanyl group includes, for example, a trifluoromethylsulfanyl group.
The halogenated alkylsulfonyl group means an alkylsulfonyl group in which one or more hydrogen atoms in the alkylsulfonyl group are substituted by halogen atoms. Examples of the halogenated alkylsulfonyl group include, for example, a halogenated C₁₋₆ alkylsulfonyl group. Moreover, the halogenated C₁₋₆ alkylsulfonyl group includes, for example, a trifluoromethylsulfonyl group.

The phenylalkyl group includes, for example, a phenyl C₁₋₆ alkyl group. Moreover, the phenyl C₁₋₆ alkyl group includes, for example, a benzyl group or a phenylethyl group.
The phenylalkoxy group includes, for example, a phenyl C₁₋₆ alkoxy group. Moreover, the phenyl C₁₋₆ alkoxy group includes, for example, a benzyloxy group.
The phenylalkenyl group includes, for example, a phenyl C₂₋₇ alkenyl group. Moreover, the phenyl C₂₋₇ alkenyl group includes, for example, a 2-phenylethen-1-yl group (a styryl group).
The phenylalkynyl group includes, for example, a phenyl C₂₋₇ alkynyl group. Moreover, the phenyl C₂₋₇ alkynyl group includes, for example a phenylethynyl group.

The alkoxycarbonyl group includes, for example, a C₁₋₆ alkoxycarbonyl group. Moreover, the C₁₋₆ alkoxycarbonyl group includes, for example, a methoxycarbonyl group.
The alkoxyiminoalkyl group includes, for example, a C₁₋₆ alkoxyimino C₁₋₆ alkyl group. Moreover, the C₁₋₆ alkoxyimino C₁₋₆ alkyl group includes, for example, a 1-methoxyiminoethyl group.
The benzyloxyiminoalkyl group includes, for example, a benzyloxyimino C₁₋₆ alkyl group. Moreover, the benzyloxyimino C₁₋₆ alkyl group includes, for example, a 1-(benzyloxyimino)ethyl group.

The trimethylsilylalkynyl group includes, for example, a trimethylsilyl C₂₋₇ alkynyl group. Moreover, the trimethylsilyl C₂₋₇ alkynyl group includes, for example, a trimethylsilylethynyl group.
The 1-(5-phenyl)-3-(halogenated alkyl)pyrazolyl group includes, for example, a 1-(5-phenyl)-3-(halogenated C₁₋₆ alkyl)pyrazolyl group. Moreover, the 1-(5-phenyl)-3-(halogenated C₁₋₆ alkyl)pyrazolyl group includes, for example, a 1-(5-phenyl)-3-(trifluoromethyl)pyrazolyl group.
The 1-(5-alkyl)-3-(halogenated alkyl)pyrazolyl group includes, for example, a 1-(5- C₁₋₆ alkyl)-3-(halogenated C₁₋₆ alkyl)pyrazolyl group. Moreover, examples of the 1-(5- C₁₋₆ alkyl)-3-(halogenated C₁₋₆ alkyl)pyrazolyl group include, for example, a 1-(5-tert-butyl)-3-(trifluoromethyl)pyrazolyl group.
The 1-[3,5-bis(halogenated alkyl)]pyrazolyl group includes, for example, a 1-[3,5-bis(halogenated C₁₋₆ alkyl)]pyrazolyl group. Moreover, examples of the 1-[3,5-bis(halogenated C₁₋₆ alkyl)]pyrazolyl group include, for example, a 1-[3,5-bis(trifluoromethyl)]pyrazolyl group.

The substance selected from the group consisting of the compound represented by the aforementioned general formula (I);the salt thereof; the hydrates thereof; and the solvates thereof is useful as an inhibitor of PAI-1 production and as a medicament for the prevention or the improvement of diseases caused by the overproduction of PAI-1.
Examples of diseases caused by the overproduction of PAI-1 include, for example, thrombus formation, fibrosis, visceral fat deposition, angiogenesis, deposition and reconstruction of extracellular matrix, diseases caused by the proliferation, migration, infiltration and metastasis of cells (for example, tumor cells, vascular endothelial cells and the like), the reconstruction of tissues (for example, heart remodeling, vascular remodeling and the like) and the like, to be more specific, hepatopathy by biliary obstruction, cerebral thrombosis, cerebrosclerosis, cerebral infarction, transient ischemic attack, stroke, cerebrovascular dementia, ischemic cerebrovascular disease, angina, atrial thrombus by atrial fibrillation, cardiac failure, ischemic heart disease, pulmonary thrombosis, pulmonary embolism, thrombotic pulmonary disease, deep-vein thrombosis (DVT), thrombophlebitis, occlusive venous disease, acute arterial occlusion, chronic arterial occlusion, occlusive peripheral arterial disease, thrombus after bypass vascular transplantation, disseminatal intravascular coagulation syndrome (DIC), acute coronary occlusion after percutaneous transluminal coronary angioplasty (PTCA), angiopathy and thrombus caused by congenital thrombus disposition such as gene abnormality, renal thrombosis, renal emphraxis, thrombotic renal disease, thrombotic disease, thrombosis, , blood coagulation, ischemic disease, heart attack, deep-seated thrombosis, venous thromboembolism, nephrosclerosis, metabolic syndrome, aldosteronic tissue disorder, failing organs, interstitial cystitis, prostate hyperplasia, amyotrophy, memory disorder, age-related macular degeneration, cystic fibrosis, hepatic fibrosis, lymphedema, dysuria, or vascular events of brain, heart and the like.

Moreover, the above medicament is useful for the prevention or improvement of diseases involving the overproduction of PAI-1, for example, disseminatal intravascular coagulation syndrome (DIG), immune disorder such as antiphospholipid antibody syndrome, arteriosclerosis, metabolic syndrome, economy class syndrome, endotoxin shock, allergic disease, vasculitis, nonbacterial thrombotic endocarditis, serious infectious disease such as sepsis, fibrin dependent pain in arthritis, retinopathy, nephropathy, neurosis, peripheral circulatory disorder, diabetic complication, hypertension, diabetes, hyperinslinemia, hypercholesterolemia, insulin resistance, hyperlipemia, obesity, tumor, tumor infiltration, tumor metastasis, asthma, rejection after implantation of heart or kidney, thrombus formation such as arterial lesion after tissue transplantation, or polycystic nephropathy. Furthermore, since the medicament of the present invention can prevent and improve thrombus formation, it is effective for wound healing and decubitus healing.

The compound represented by the general formula (I) used in the present invention can be prepared by the methods described in documents, for example, International Publication No 99/65449 Pamphlet, International Publication No 02/049632 Pamphlet, International Publication No 03/103654 Pamphlet, and International Publication No 03/103658. Furthermore, salts of the compound represented by the general formula (I), and the hydrates or solvates of the salt or the compound represented by the general formula (I) can be prepared by the conventional methods in the art. As the salts of the compound represented by the general formula (I), it is preferable to use pharmacologically acceptable salts of the aforementioned compound. Examples of the aforementioned salts include, for example, alkali metal salts, alkaline earth metal salts, other metal salts, inorganic salts, and organic salts.

The inhibitor of PAI-1 production or the medicament of the present invention may consist of only the compound represented by the general formula (I) or pharmacologically acceptable salts thereof, or hydrates or solvates thereof, and it may be a composition further containing one or more than two pharmacologically acceptable pharmaceutical additives. Examples of aforementioned pharmaceutical additives include existing additives, for example, excipient, binder, lubricant, disintegrator, corrigent, solvent, stabilizer, base material, wetting agent, and preservative. The medicament of the present invention may be in any dosage form, for example, tablet, capsule, granule, powder, solution, subtle granules, powdered medicine, syrup, injection, liniment, propellant, patch, and suppository. Furthermore, the medicament of the present invention may be administered in any means, examples of the means include oral administration, and parenteral administration such as intravenous administration.

### [Examples]

In the following, the present invention is explained more specifically with examples; however, the scope of the present invention is not limited to the following examples. As the test substances of Example 5, each compound represented by the general formula (II) in which R¹ to R¹⁰ of the compound are the substituents listed in the following table was used. In the Table, Me represents a methyl, t-Bu represents a tert-butyl, n-Hex represents an n-butyl, i-Pr represents an isopropyl, and sec-Bu represents a sec-butyl, respectively.

**[Table1-1]**

| Compound | R¹ | R² | R³ | R⁴ | R⁵ | R⁶ | R⁷ | R⁸ | R⁹ | R¹⁰ |
|---|---|---|---|---|---|---|---|---|---|---|
| (2) | H | H | H | Cl | H | H | CF₃ | H | CF₃ | H |
| (3) | H | H | H | Br | H | CF₃ | H | H | CF₃ | H |
| (4) | H | H | H | H | H | H | CF₃ | H | CF₃ | H |
| (5) | H | H | H | F | H | H | CF₃ | H | CF₃ | H |
| (6) | H | H | H | Br | H | H | CF₃ | H | CF₃ | H |
| (7) | H | H | H | I | H | H | CF₃ | H | CF₃ | H |
| (8) | H | H | H | NO₂ | H | H | CF₃ | H | CF₃ | H |
| (9) | H | H | H | CN | H | H | CF₃ | H | CF₃ | H |
| (10) | H | H | H | Me | H | H | CF₃ | H | CF₃ | H |
| (11) | H | H | H | t-Bu | H | H | CF₃ | H | CF₃ | H |
| (12) | H | H | H | 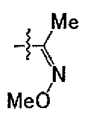 | H | H | CF₃ | H | CF₃ | H |
| (13) | H | H | H | 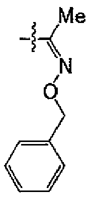 | H | H | CF₃ | H | CF₃ | H |
| (14) | H | H | H | 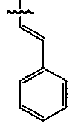 | H | H | CF₃ | H | CF₃ | H |
| (15) | H | H | H | 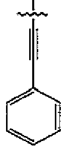 | H | H | CF₃ | H | CF₃ | H |
| (16) | H | H | H | 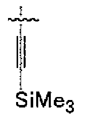 | H | H | CF₃ | H | CF₃ | H |
| (17) | H | H | H | 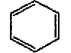 | H | H | CF₃ | H | CF₃ | H |
| (18) | H | H | H | 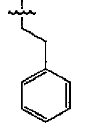 | H | H | CF₃ | H | CF₃ | H |
| (19) | H | H | H | CF₃ | H | H | CF₃ | H | CF₃ | H |

**[Table 1-2]**

| | | | | | | | | | | |
|---|---|---|---|---|---|---|---|---|---|---|
| (20) | H | H | H | CF₂CF₃ | H | H | CF₃ | H | CF₃ | H |
| (21) | H | H | H | 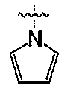 | H | H | CF₃ | H | CF₃ | H |
| (22) | H | H | H | 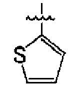 | H | H | CF₃ | H | CF₃ | H |
| (23) | H | H | H | 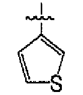 | H | H | CF₃ | H | CF₃ | H |
| (24) | H | H | H | 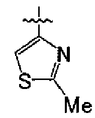 | H | H | CF₃ | H | CF₃ | H |
| (25) | H | H | H | 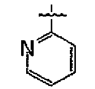 | H | H | CF₃ | H | CF₃ | H |
| (26) | H | H | H | C(O)Me | H | H | CF₃ | H | CF₃ | H |
| (27) | H | H | H | 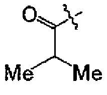 | H | H | CF₃ | H | CF₃ | H |
| (28) | H | H | H | CO₂Me | H | H | CF₃ | H | CF₃ | H |
| (29) | H | H | H | 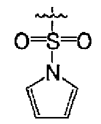 | H | H | CF₃ | H | CF₃ | H |
| (30) | H | H | H | 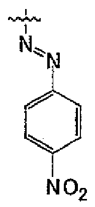 | H | H | CF₃ | H | CF₃ | H |
| (31) | C(O)Me | H | H | H | H | H | CF₃ | H | CF₃ | H |
| (32) | C(O)Me | H | H | Cl | H | H | CF₃ | H | CF₃ | H |
| (33) | H | H | Cl | H | H | H | CF₃ | H | CF₃ | H |
| (34) | H | H | H | Cl | H | H | H | Cl | H | CF₃ |
| (35) | H | H | H | Cl | H | H | H | H | CF₃ | F |
| (36) | H | H | H | Cl | H | H | H | F | CF₃ | H |
| (37) | H | H | H | Cl | H | H | F | H | CF₃ | H |
| (38) | H | H | H | Br | H | Cl | H | H | CF₃ | H |
| (39) | H | H | H | Br | H | H | H | CN | CF₃ | H |
| (40) | H | H | H | Cl | H | H | H | H | CF₃ | Me |
| (41) | H | H | H | Br | H | H | OMe | H | CF₃ | H |

**[Table1-3]**

| | | | | | | | | | | |
|---|---|---|---|---|---|---|---|---|---|---|
| (42) | H | H | H | Cl | H | SMe | H | H | CF₃ | H |
| (43) | H | H | H | Cl | H | H | H | CF₃ | H | H |
| (44) | H | H | H | Br | H | H | H | H | Cl | H |
| (45) | H | H | H | Br | H | H | H | Cl | H | H |
| (46) | H | H | H | Br | H | H | F | H | F | H |
| (47) | H | H | H | H | H | H | Cl | H | Cl | H |
| (48) | H | H | Cl | H | H | H | Cl | H | Cl | H |
| (49) | H | H | H | Br | H | H | Cl | Cl | Cl | H |
| (50) | H | H | H | Br | H | H | Me | H | Me | H |
| (51) | H | H | H | Cl | H | H | t-Bu | H | t-Bu | H |
| (52) | H | H | H | Cl | H | H | H | H | 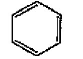 | H |
| (53) | H | H | H | Cl | H | H | OMe | H | 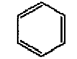 | H |
| (54) | C(O)Me | H | H | H | H | H | Cl | H | Cl | H |
| (55) | H | H | H | H | OH | H | CF₃ | H | CF₃ | H |
| (56) | H | H | Me | H | H | H | CF₃ | H | CF₃ | H |
| (57) | H | Cl | H | Cl | H | H | CF₃ | H | CF₃ | H |
| (58) | H | H | H | 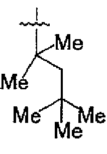 | H | H | CF₃ | H | CF₃ | H |
| (59) | H | Cl | H | Cl | Cl | H | CF₃ | H | CF₃ | H |
| (60) | H | H | H | H | F | H | CF₃ | H | CF₃ | H |
| (61) | H | Cl | H | H | H | H | CF₃ | H | CF₃ | H |
| (62) | H | H | H | Cl | H | 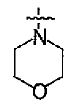 | H | H | CF₃ | H |
| (63) | H | H | H | Cl | H | H | OCH₂CF₃ | H | CF₃ | H |
| (64) | H | H | H | Cl | H | O-n-Hex | H | H | SO₂Me | H |
| (65) | H | H | H | Cl | H | Me | H | H | C(O)-t-Bu | H |
| (66) | H | H | H | Cl | H | Me | H | H | 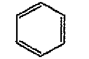 | H |
| (67) | H | H | H | Cl | H | H | CF₃ | H | H | H |
| (68) | H | H | H | Cl | H | H | OCF₃ | H | H | H |
| (69) | H | H | H | Cl | H | 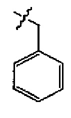 | H | H | H | H |

**[Table1-4]**

| | | | | | | | | | | |
|---|---|---|---|---|---|---|---|---|---|---|
| (70) | H | H | H | Cl | H | H | H | OCF₃ | H | H |
| (71) | H | H | H | Cl | H | Cl | H | Cl | H | H |
| (72) | H | H | H | Cl | H | H | H | t-Bu | H | H |
| (73) | H | H | H | Cl | H | H | H | n-Hex | H | H |
| (74) | H | H | H | Cl | H | H | H | 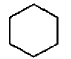 | H | H |
| (75) | H | H | H | Cl | H | H | H | 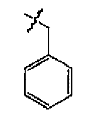 | H | H |
| (76) | H | H | H | Cl | H | Cl | Cl | H | H | H |
| (77) | H | H | H | Cl | H | Cl | H | H | H | H |
| (78) | H | H | H | Cl | H | H | H | n-Bu | H | H |
| (79) | H | H | H | Cl | H | H | H | 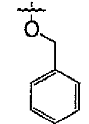 | H | H |
| (80) | H | H | H | Cl | H | H | 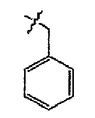 | H | H | H |
| (81) | H | H | H | Cl | H | H | i-Pr | H | H | H |
| (82) | H | H | H | Cl | H | H | SCF₃ | H | H | H |
| (83) | H | H | H | Cl | H | H | H | SCF₃ | H | H |
| (84) | H | H | H | Cl | H | H | H | SO₂CF₃ | H | H |
| (85) | H | H | H | Cl | H | H | F | F | H | H |
| (86) | H | H | H | Cl | H | H | 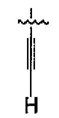 | H | H | H |
| (87) | H | H | H | Cl | H | H | H | sec-Bu | H | H |
| (88) | H | H | H | Cl | H | H | 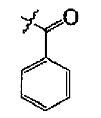 | H | H | H |
| (89) | H | H | H | Cl | H | H | H | 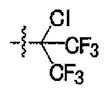 | H | H |
| (90) | H | H | H | Cl | H | H | H | O-CHF₂ | H | H |
| (91) | H | H | H | Cl | H | H | 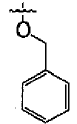 | H | H | H |
| (92) | H | H | H | Cl | H | H | t-Bu | H | H | H |

**[Table1-5]**

| | | | | | | | | | | |
|---|---|---|---|---|---|---|---|---|---|---|
| (93) | H | H | H | Cl | H | H | 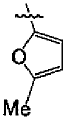 | H | H | H |
| (94) | H | H | H | Cl | H | H | SO₂CF₃ | H | H | H |
| (95) | H | H | H | Cl | H | H | O-n-Hex | H | H | H |
| (96) | H | H | H | Cl | H | H | H | 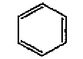 | H | H |
| (97) | H | 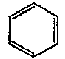 | H | H | H | H | OF₃ | H | CF₃ | H |
| (98) | H | i-Pr | H | H | H | H | CF₃ | H | CF₃ | H |
| (99) | H | i-Pr | H | Br | H | H | CF₃ | H | CF₃ | H |
| (100) | H | Me | H | Br | H | H | CF₃ | H | CF₃ | H |
| (101) | H | H | H | Cl | H | H | 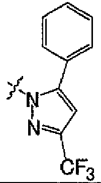 | H | H | H |
| (102) | H | H | H | Cl | H | H | H | 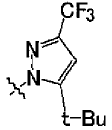 | H | H |
| (103) | H | 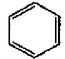 | H | Br | H | H | CF₃ | H | CF₃ | H |
| (104) | H | H | H | Cl | H | H | H | 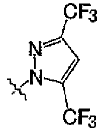 | H | H |
| (105) | H | H | H | Cl | H | H | 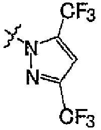 | H | H | H |
| (106) | H | H | 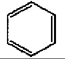 | H | H | H | CF₃ | H | CF₃ | H |
| (107) | H | H | Cl | H | H | H | H | 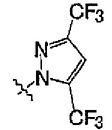 | H | H |
| (108) | H | H | H | Cl | H | H | H | 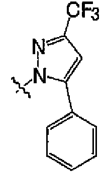 | H | H |

### Example 1: Inhibitory effect of the compound (1) on PAI-1 production

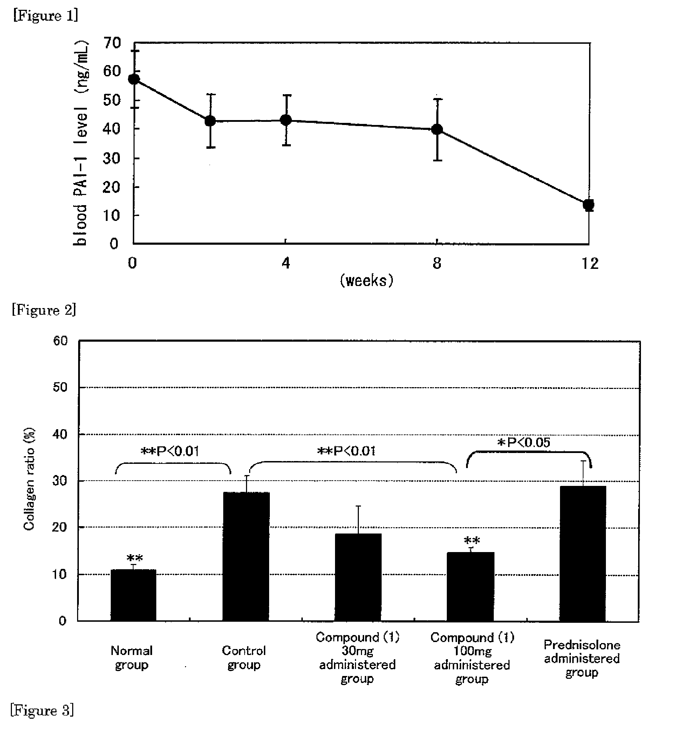

To 10 patients of the type II diabetes with obesity where PAI-1 overproduction was recognized, the compound (1) was administered orally 200 mg each time twice a day (after breakfast and after dinner) for 12 weeks. Blood was collected from each patient just before the administration of the compound (1) and 2, 4, 8, and 12 weeks later, and the PAI-1 concentration in plasma was measured using Human PAI-1 ELISA Kit (AssayPro). The results are shown in Fig. 1.
As shown in Fig. 1, since the concentration of PAI-1 in blood decreased by the administration of the compound (1), it was found out that the compound (1) has an inhibitory action on the production of abnormally enhanced PAI-1. Consequently, the compound (1) has an anti-blood coagulation activity and an anti-thrombotic activity and it is suggested that the compound can improve thrombophilia.

### Example 2: Inhibitory effect of the compound (1) on collagen deposition

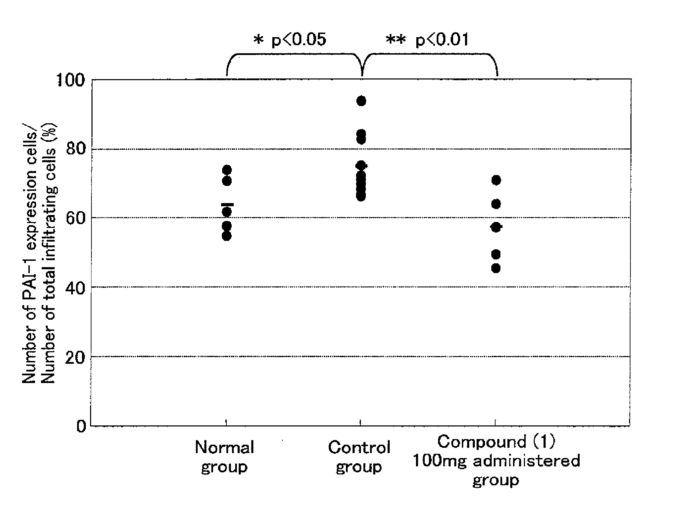

Using the smoking exposure system (INH06-CIG01A, MIPS Inc.), 6-week old guinea pigs (Std: male Hartley, Japan SLC, Inc.) were exposed to smoke of 40 cigarettes (Peace (Registered Trademark), Japan Tobacco, Inc.) a day (20 cigarettes twice) for 25 continuous days (Chronic Obstructive Pulmonary Disease (COPD) model group: Control group: n=10). To theseguinea pigs, from the day of exposure to the smoke of tobacco, the compound (1) (30 mg/kg/day or 100 mg/kg/day) or prednisolone (10 mg/kg/day) was continuously administered orally once a day for 25 days (Compound (1) 30 mg administered group: n=5, Compound (1) 100 mg administered group: n=5, Prednisolone administered group: n=5). Furthermore, as a normal group, guinea pigs (n=5) exposed to air without the administration of the compound (1) and prednisolone were prepared. On the following day of the last exposure, after preparing paraffin sections in the lungs of each guinea pig, collagen was stained by the Masson-trichrome method, and the accumulations of collagen in the alveoli were measured by the area rate with the image analysis. The results are shown in Fig.2.
As shown in Fig.2, prednisolone that is a steroid was not able to inhibit the accumulation of collagen. On the other hand, the compound (1) was able to inhibit the accumulation of collagen. Consequently, it was found out that the compound (1) has an inhibitory activity on the accumulation of collagen, and is effective for the inhibition of fibrosis. Since these results well agree with the fact that anti-inflammatory steroid does not inhibit PAI-1 production, this action is presumed to be attributed to the inhibition of the production of PAI-1.
Next, the above paraffin section was stained by the immunostaining method using an anti PAI-1 antibody (PAI-1 H-135 ; Santa Cruz ; 250-fold dilution), and the ratio of PAI-1 expression cells to the total cell numbers infiltrated in the lung was measured. The immunostaining was carried out using the IHC Select HRP/DAB (CHEMICON). The results are shown in Fig. 3.
As shown in Fig.3, it was demonstrated that the ratio of the PAI-1 expression cells to the total number of the infiltrating cells decreased by the administration of the compound (1). Furthermore, since the total number of the infiltrating cells in the normal group and the compound (1) 100 mg administered group were at the same level, it was demonstrated that the ratio of the PAI-1 expression cells to the total number of the infiltration cells improved to the level of the normal group by the administration of the compound (1). From the above results, it was suggested that the inhibition of collagen accumulation by the compound (1) was related to the inhibition of PAI-1 production.

### Example 3: Inhibitory effect of the compound (1) on fibrosis

After the 8-week old rats (SPF: male SD, Japan SLC, Inc.) were anesthetized with Pentobarbital Sodium, their hearts were exteriorized, and the left anterior descending coronary arteries (LAD) were ligated by a suture needle with a thread. 30 minutes later, the ligations were removed, and the blood was reperfused for 24 hours. After that, the compound (1) (10 mg/kg/day, 30 mg/kg/day, 100 mg/kg/day) was orally administered once a day for 28 continuous days. Moreover, as a control, rats to which the compound (1) was not administered after the reperfusion (cardiac infarction (ischemia-reperfusion) model rats) were prepared. On the 29^{th} day after the reperfusion, after preparing paraffin sections of cardiac muscle in the left ventricle of each rat, Azan-Mallory stain was carried out, the ratios of the stained area occupying the myocardial necrosis distant sites were obtained as the ratios of fibrosis by the image analyses. The results are shown in the following Table.

**[Table 2]**

| Administration group | Dose (mg/kg, p.o.) | Number of animals | Fibrosis ratio (%) |
|---|---|---|---|
| Control | - | 10 | 7.5±1.1 |
| (Compound(1) | 10 | 9 | 5.5±0.4 |
| | 30 | 10 | 4.9±0.4 |
| | 100 | 10 | 4.3±0.5 |

As shown on the Table, the compound (1) inhibited fibrosis effectively. From this fact, it is revealed that the compound (1) is useful for the inhibition of heart remodeling.

### Example 4: Inhibitory effect of the compound (I) on fibrosis

A polyethylene tube was inserted to the urethra of the 7 week-old rats (SPF: male Crj, Charles River Japan, Inc), and 0.4 mL of 5% mustard oil (diluted with liquid paraffin including 20% allyl isothiocyanate) was infused into the bladder. 60 minutes later, after withdrawing the liquid in the bladder with suctioning, the inside of the bladder was rinsed with saline. For 10 days including 3 days before the infusion of the mustard oil and 7 days after the infusion of the mustard oil, the compound (1) (100 mg/kg/day) was orally administered once a day (Compound (1) administered rat: n=6). Furthermore, as a control, mustard oil infusion rats (cystitis rat: n=6) to which the compound (1) was not administered were prepared. On the 8^{th} day after the infusion of the mustard, the bladders were removed to prepare paraffin sections, the degrees of fibrosis were scored by Azan stain, and the evaluations were conducted. For the index of the scoring, those that have no abnormalities to the normal tissues being "1," those that have minor fibrosis being "2," those that have mild fibrosis being "3," those that have moderate fibrosis being "4," and those that have severe fibrosis being "5" were used. As a result, although the score of the control group was 2.6±0.1, the compound (1) administered group was 2.0±0.2, and a significant difference was confirmed (p<0.01: Wilcoxon test). From this result, it was found out that the the compound (1) is useful for the inhibition of fibrosis.

### Example 5: Inhibitory effect of the test substances on TGF-β induced PAI-1 production

After incubating HT1080 cells for 48 hours in the MEM medium containing 10%FBS (Fetal Bovine Serum), the medium was changed to a MEM medium containing 1% FBS, and TGF-β1 (final concentration: 10 ng/mL) only, or TGF-β1 (final concentration: 10 ng/mL) and the test substances were added and incubated. As a control, a culture solution without neither TGF-β1 nor the test substances was prepared. After 6-hours incubation, the culture fluids were collected, and the PAI-1 amounts in the culture fluids were measured by the Western blotting. The PAI-1 amount when TGF-β1 only was added being "100%," and the PAI-1 amount in the control being "0%," PAI-1 I amounts at each concentration of the test substances (PAI-1 inhibition ratios) were calculated, and the concentrations of the compounds, which inhibited PAI-1 production to 50% (IC₅₀) in amount, were obtained. The results are shown in the following Table.

**[Table 3]**

| Compound Number | IC50 (nM) |
|---|---|
| (2) | 5 |
| (3) | 83 |

### Example 6: Inhibitory effect of the test substances on TGF-β induced PAI-1 production

HepG2 cells were seeded on a 96-well culture plate by 2x10⁴ cells/well, and were incubated for 24 hours in the MEM medium containing 1% NEAA (Non-Essential Amino Acids), 1mM pyruvate, and 10% FBS. Then, the medium was changed to a MEM medium containing 1% NEAA, 1mM pyruvate and 1% FBS, and the cells were further incubated for 24 hours. Subsequently, after the medium was changed to a MEM medium containing 1% NEAA, 1mM pyruvate and 1% FBS, and then the cells were treated with the test substances (final concentration: 1 µM or 0.1 µM) for one hour, TGF-β1 (final concentration.1ng/mL) was added and the cells were further incubated for 24 hours. As controls, a culture solution added TGF-β1 only and a culture solution cultured without neither the test substances nor TGF-β1 were prepared. After the incubation, the PAI-1 amount was measured using Human Serpin E1/PAI-1 DuoSet (R&D Systems). The PAI-1 amount when treated with TGF-β1 only being "100%", and the PAI-1 amount when treated without neither the test substances nor TGF-β1 being "0%", the PAI-1 inhibition ratios of each test substance were calculated. The results are shown in the following two tables. IC₅₀ of the compound (2) was about 50 nM.

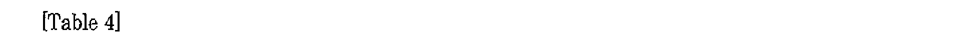

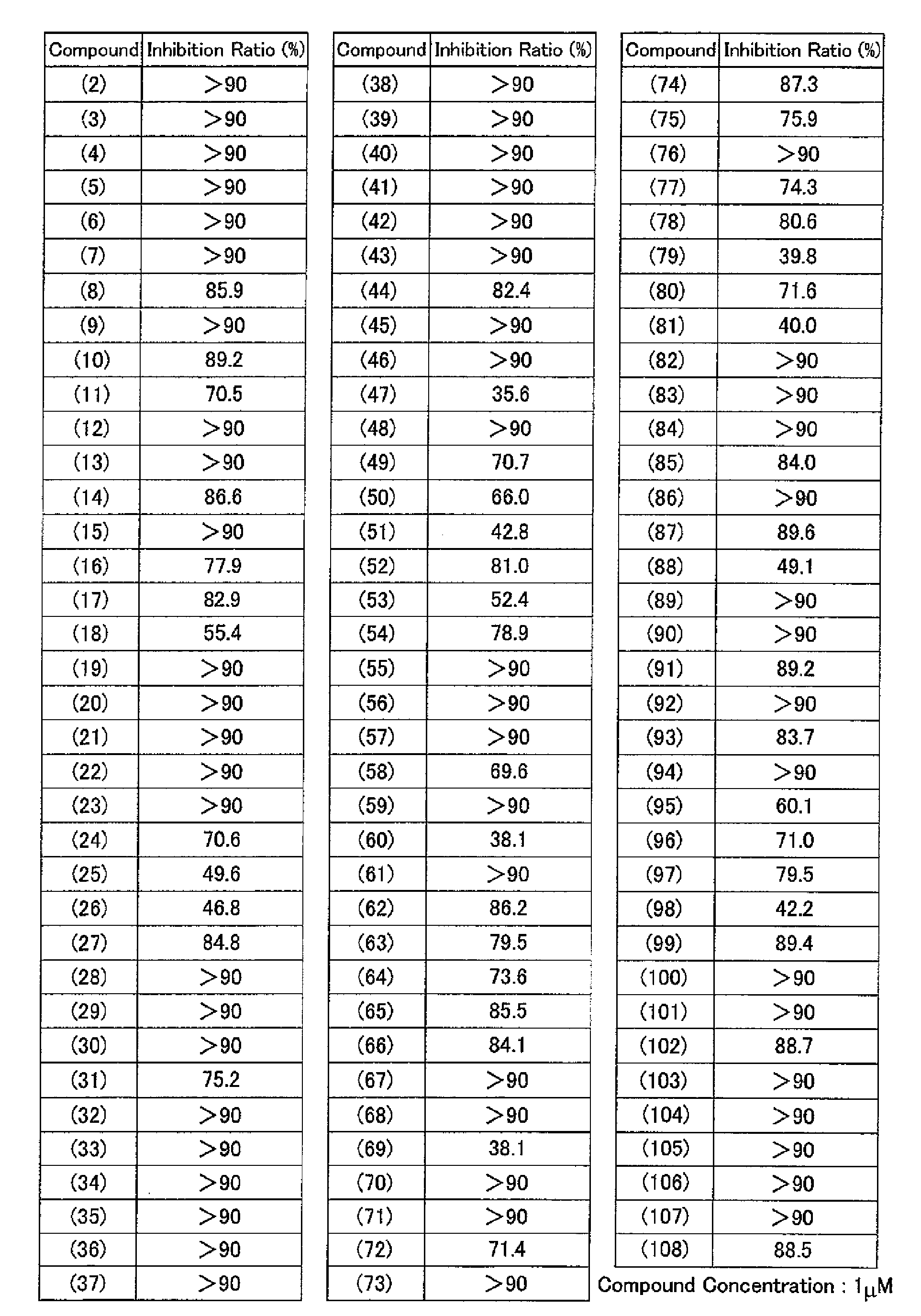

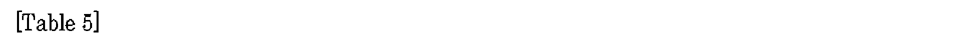

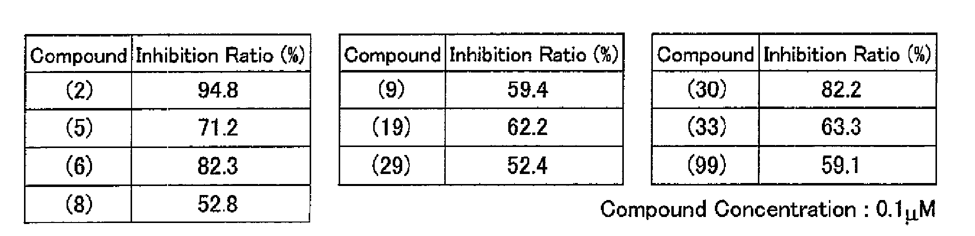

### [Industrial Applicability]

By the present invention, it became possible to provide novel substances that inhibit PAI-1 production and are useful for inhibiting blood coagulation or thrombus formation, and uses thereof.

## Claims

1. A method of inhibiting PAI-1 production, comprising the step of allowing a compound represented by the following general formula (I) or a salt thereof to act on a PAI-1 production cell, 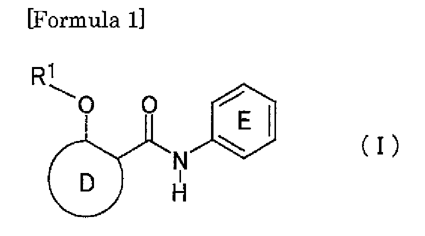 wherein
R¹ represents a hydrogen atom, an alkylcarbonyl group, or a 4-(morpholinyl)carbonyl group; D represents a benzene ring, or a benzene ring having one or more groups selected from the group consisting of a halogen atom, a nitro group, a cyano group, an alkyl group, an alkoxyiminoalkyl group, a benzyloxyiminoalkyl group, a phenylalkenyl group, a phenylalkynyl group, a trimethylsilylalkynyl group, a phenyl group, a phenylalkyl group, a halogenated alkyl group, a pyrrolyl group, an alkylpyrrolyl group, a thienyl group, an alkylthienyl group, an alkylthiazolyl group, a pyridyl group, an alkylpyridyl group, an alkylcarbonyl group, an alkoxycarbonyl group, a pyrrolylsulfonyl group, a (4-nitrophenyl)diazenyl group and a hydroxyl group; and
E is a phenyl group substituted by one or more groups selected from the group consisting of a halogen atom, a halogenated alkyl group, a phenyl group, an alkyl group, a halogenated alkoxy group, a phenylalkyl group, an alkoxy group, a cyclohexyl group, a phenylalkoxy group, a cyano group, a halogenated alkylsulfanyl group, a halogenated alkylsulfonyl group, an ethynyl group, a benzoyl group, an alkylsulfanyl group, an alkylsulfonyl group, a morpholino group, an alkylfuryl group, an alkylcarbonyl group, a 1-(5-phenyl)-3-(halogenated alkyl)pyrazolyl group, a 1-(5-alkyl)-3-(halogenated alkyl)pyrazolyl group, and a 1-[3,5-bis(halogenated alkyl)]pyrazolyl group.

2. The method according to claim 1, wherein
R¹ is a hydrogen atom, a C₁₋₆ alkylcarbonyl group, or a 4-(morphonyl)carbonyl group,
D is a benzene ring, or a benzene ring having one or more groups selected from the group consisting of a halogen atom, a nitro group, a cyano group, a C₁₋₁₀ alkyl group, a C₁₋₆ alkoxyimino C₁₋₆ alkyl group, a benzyloxyimino C₁₋₆ alkyl group, a phenyl C₂₋₆ alkenyl group, a phenyl C₂₋₆ alkynyl group, a trimethylsilyl C₂₋₈ alkynyl group, a phenyl group, a phenyl C₁₋₆ alkyl group, a halogenated C₁₋₆ alkyl group, a pyrrolyl group, a C₁₋₆ alkylpyrrolyl group, a thienyl group, a C₁₋₆ alkylthienyl group, a C₁₋₆ alkylthiazolyl group, a pyridyl group, a C₁₋₆ alkylpyridyl group, a C₁₋₆ alkylcarbonyl group, a C₁₋₆ alkoxycarbonyl group, a pyrrolylsulfonyl group, a (4-nitrophenyl)diazenyl group, and a hydroxyl group,
E is a phenyl group substituted by one or more groups selected from the group consisting of a halogen atom, a halogenated C₁₋₆ alkyl group, a phenyl group, a C₁₋₆ alkyl group, a halogenated C₁₋₆ alkoxy group, a phenyl C₁₋₆ alkyl group, a C₁₋₆ alkoxy group, a cyclohexyl group, a phenyl C₁₋₆ alkoxy group, a cyano group, a halogenated C₁₋₆ alkylsulfanyl group, a halogenated C₁₋₆ alkylsulfonyl group, an ethynyl group, a benzoyl group, a C₁₋₆ alkylsulfanyl group, a C₁₋₆ alkylsulfonyl group, a morpholino group, a C₁₋₆ alkylfuryl group, a C₁₋₆ alkylcarbonyl group, a 1-(5-phenyl)-3-(halogenated C₁₋₆ alkyl pyrazolyl group, a 1-(5- C₁₋₆ alkyl)-3-(halogenated C₁₋₆ alkyl)pyrazolyl group, and a 1-[3,5-bis(halogenated C₁₋₆ alkyl)]pyrazolyl group.

3. The method according to claim 1, wherein said compound is a compound represented by any one of the following formulas (1) to (3).

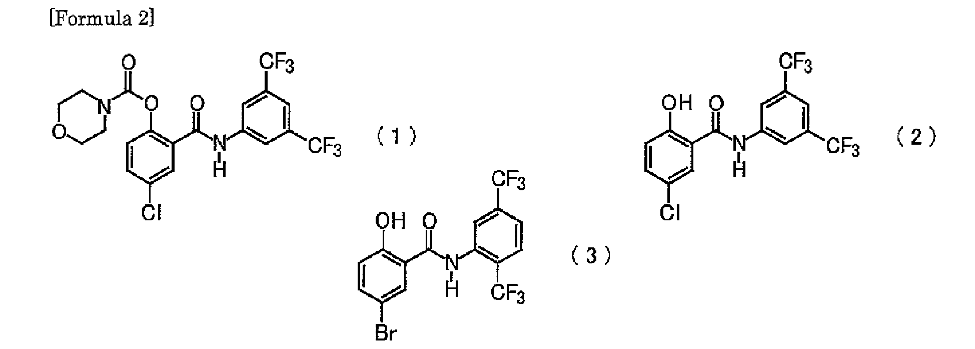

4. A method for prevention or improvement of a disease caused by an overproduction of PAI-1, comprising the step of administering an effective dose of the compound according to claim 1 or the salt thereof to a mammal including a human.
